# NEUE EUROPÄISCHE PATENTSCHRIFT

(11) **EP 1 935 375 B2**
(45) Veröffentlichungstag und Bekanntmachung des Hinweises auf die Entscheidung über den Einspruch: **23.12.2015**
(45) Hinweis auf die Patenterteilung: 10.03.2010
(21) Anmeldenummer: 07024710.1
(22) Anmeldetag: 20.12.2007
(51) Int. Cl.: A61F 2/06

(54) **Gewebte Aortensinusprothese mit Bulbus**
Woven aortic sinus prosthesis with bulb
Prothèse tissulaire de sinus aortique avec bulbe

(30) Priorität: 22.12.2006 DE 102006062360; 13.03.2007 DE 102007013428; 19.10.2007 WO PCT/EP2007/009080
(43) Veröffentlichungstag der Anmeldung: 25.06.2008
(73) Patentinhaber: Aesculap AG, 78532 Tuttlingen (DE)
(72) Erfinder: Goldmann, Helmut, 34119 Kassel (DE); Merckle, Christof, 68199 Mannheim (DE); Sievers, Hans-Hinrich, 24119 Kronshagen (DE)
(74) Vertreter: Patentanwälte Ruff, Wilhelm, Beier, Dauster & Partner mbB

(56) Entgegenhaltungen:
- EP-A- 0 955 019
- WO-A-99/40875
- WO-A-2005/099624
- JP-A- 3 045 743
- US-A- 4 346 741
- US-A- 5 139 515
- US-A- 5 662 675
- US-A- 6 053 938
- US-A1- 2003 078 650
- US-A1- 2005 240 261
- US-B1- 6 454 796
- Anderson, Kim "Seamless Textiles with Inherent Shape", Raleigh, NC: North Carolina state University, 2004

## Beschreibung

Die vorliegende Erfindung betrifft eine gewebte Aortensinusprothese.

Der Ersatz von herznahen Bereichen der Aorta, insbesondere der sogenannten Aorta ascendens, zählt zu den wichtigsten Aufgabenfeldern der modernen Herzchirurgie. Hierfür stehen in der Zwischenzeit eine Vielzahl von Gefäßprothesen zur Verfügung. Die Gefäßprothesen werden gewöhnlich nach Entfernung von aneurysmatischen Teilen der Aorta ascendens und insbesondere nach Entfernung der Aortensinus (natürlichen Sinus) unter Erhalt der Aortenklappen und Teilen der Arterienwand implantiert.

Bei den zuerst hergestellten Gefäßprothesen handelte es sich um gerade und homogene Rohrprothesen. Nachteilig bei derartigen Gefäßprothesen ist, dass ihnen zusätzliche Strukturen fehlen, welche insbesondere die Funktion von operativ entfernten Aortensinus ersetzen könnten. Demnach spiegeln sie die tatsächlichen anatomischen Verhältnisse der Aorta ascendens nur unzureichend wieder.

Aus der US 6,544,285 B1 sowie der WO 01152776 A1 sind röhrenförmige Gefäßprothesen für den Ersatz der Aorta ascendens bekannt, welche im herznahen Bereich künstliche Sinus zum Ersatz der Aortensinus aufweisen. Die künstlichen Sinus sind an die Prothese angenäht. Durch die angenähten Sinus entstehen am herznahen Ende der Gefäßprothese bogenförmige Ausbuchtungen. Zwar erlauben diese Gefäßprothesen grundsätzlich eine verbesserte Abbildung der tatsächlichen anatomischen Gegebenheiten der Aorta ascendens, doch gestaltet sich ihre Implantation vielfach aufwendig.

Die EP 0 955 019 A2 betrifft röhrenförmige Gefäßprothesen, welche im Bereich des Aortenbulbus eine generelle circumferentielle Ausbuchtung aufweisen. Auch diese Prothesenkonfiguration entspricht nicht der tatsächlich vorliegenden Anatomie der Aorta ascendens. Eine in dieser Hinsicht deutlich verbesserte röhrenförmige Gefäßprothese wird in der WO 2004/021925 A2 beschrieben, wonach die Röhre im Bereich der Aorta ascendens mindestens zwei künstliche Sinus aufweist, die eine größere Ausdehnung nach außen besitzen als die Röhre selbst. Weiterhin wird in der US 5,139,515 eine Aortenprothese mit sinusartigen Ausbuchtungen beschrieben, ohne allerdings nähere Einzelheiten zur Ausbildung der Ausbuchtungen zu offenbaren. Die WO 2005/099624 A1 beschreibt eine gewebte Aortensinusprothese mit einem im wesentlichen kugelförmigen Bulbusabschnitt. Zur Herstellung ist eine komplizierte Webtechnik erforderlich. Aus der JP 030 457 43 A gehen schlauchförmige Textilien mit unterschiedlich voneinander beabstandeten Kettfäden hervor. Ferner sind gewebte Gefäßprtothesen mit schlauchförmigen Ausbuchtungen bekannt (Kim Anderson, Dissertation 2005, North Carolina State University).

Aufgabe der vorliegenden Erfindung ist es, eine einfach herzustellende Gefäßprothese zum Ersatz der Aorta ascendens bereitzustellen, welche aus dem Stand der Technik bekannte Nachteile vermeidet und insbesondere eine Konfiguration besitzt, welche die natürlichen anatomischen Gegebenheiten der Aorta ascendens in zufriedenstellender Weise wiederspiegelt.

Diese Aufgabe wird erfindungsgemäß gelöst durch eine gewebte Aortensinusprothese gemäß Anspruch 1.

Durch die Erfindung wird eine Aortensinusprothese bereitgestellt, welche sich insbesondere zum Ersatz der Aorta ascendens eignet. Durch den bulbusförmigen zweiten Abschnitt entspricht die Prothese in besonderem Maße den anatomischen Gegebenheiten der natürlichen Aorta, insbesondere mit Hinblick auf die natürlichen Sinus. So kann sich der Bulbus nach Implantation der Aortensinusprothese während der Systole mit Blut füllen, welches während der Diastole wegen der vorzugsweise elastischen Eigenschaften der Prothese weiterbefördert wird. Konstante Zahl an Kettfäden bedeutet, dass die Kettfäden über die gesamte Länge der Prothese durchgehen, ohne dass Fäden aufgenommen und/oder abgenommen sind.

In einer bevorzugten Ausführungsform weist der Bulbusabschnitt einen maximalen Durchmesser auf, der 1 bis 45 %, vorzugsweise 10 bis 40 %, insbesondere 15 bis 35 %, größer ist als der Durchmesser von zylindrischen Abschnitten der erfindungsgemäßen Aortensinusprothese. In speziellen Fällen kann der Bulbusdurchmesser um bis zu 50 % größer sein als der Durchmesser des zylindrischen Abschnitts. Normalerweise beträgt der Durchmesser der Aortensinusprothese in den zylindrischen Bereichen 24 bis 40 mm, in der Regel 28 bis 32 mm, und im Bulbusbereich maximal 30 bis 50 mm, in der Regel maximal 32 bis 48 mm, insbesondere maximal 34 bis 38 mm.

Der erste, herzferne zylindrische Abschnitt besitzt bevorzugt eine Länge von 20 bis 150 mm, insbesondere von 50 bis 100 mm und in Spezialfällen bis 200 mm. Der Bulbus besitzt vorzugsweise eine axiale Länge von 20 bis 50 mm, insbesondere von 25 bis 30 mm, in Spezialfällen bis zu 60 mm.

In einer weiteren Ausführungsform besitzt der dritte, im wesentlichen zylindrische, herznahe Prothesenabschnitt eine axiale Länge von 5 bis 30 mm, insbesondere von 10 bis 20 mm. In dieser Ausführungsform eignet sich die erfindungsgemäße Gefäßprothese zusätzlich zum Ersatz der Aorta ascendens auch zum Remodelling der Aortensinuswurzel (Annulus).

Der Bulbusabschnitt besitzt in axialer Richtung vorzugsweise drei Längsunterabschnitte, von denen ein erster, herzferner Unterabschnitt eine stetige, insbesondere kontinuierliche Abstandsvergrößerung der Kettfäden aufweist, ein zweiter, mittlerer Unterabschnitt im wesentlichen konstante Abstände der Kettfäden aufweist und ein dritter, herznaher Unterabschnitt eine stetige Verjüngung der Abstände der Kettfäden aufweist bis zum Durchmesser des zylindrischen Abschnittes der Prothese. In einer möglichen Ausführungsform besitzt der Bulbusabschnitt in axialer Richtung zwei Längsunterabschnitte, wobei sich an einen ersten, herzfernen Unterabschnitt mit einer stetigen, insbesondere kontinuierlichen Abstandsvergrößerung der Kettfäden direkt ein zweiter, herznaher Unterabschnitt mit einer stetigen Verjüngung der Abstände der Kettfäden anschließt. Erfindungsgemäß ist es insbesondere vorgesehen, dass die drei bzw. zwei Bulbusunterabschnitte in axialer Richtung verschieden lang ausgebildet sind.

In einer weitergehenden Ausführungsform ist der erste Bulbusunterabschnitt mindestens gleich lang wie ein dritter Unterabschnitt. Bevorzugt ist der erste Bulbusunterabschnitt länger als ein dritter Abschnitt. In diesem Fall verläuft die Durchmesservergrößerung des ersten Abschnittes entsprechend allmählicher. Entsprechendes gilt für Konizität des ersten Abschnittes in diesem Fall.

Die Kettfäden besitzen im Bulbusabschnitt im Querschnitt unterschiedlich große Abstände voneinander. So kann der Bulbusabschnitt bei einer Ausführungsform drei bis sechs Einzelbulbi aufweisen, die durch eine entsprechende Anzahl von Umfangsbereichen mit vergrößerten Abständen von Kettfäden gebildet werden. Die Kettfäden zwischen den Einzelbulbi können entweder im Vergleich zu den zylindrischen Prothesenabschnitten unveränderte Kettfädenabstände besitzen oder vergrößerte Abstände, die aber kleiner sind als bei den Bäuchen der Einzelbulbi. Die Abstandsvergrößerung kann so gestaltet sein, dass der Bulbusabschnitt bzw. die einzelnen Bulbi in einzelne Unterabschnitte unterteilt sind oder einen gleichmäßigen ballonförmigen Übergang besitzen.

Als Materialien zur Ausbildung der gewebten Aortensinusprothese kommen grundsätzlich alle natürlichen und/oder synthetischen Fäden in Betracht. Die Fäden können Einzelfilamente oder Multifilamente sein. Bevorzugt handelt es sich bei den Fäden um Garne. Die Fäden sind zweckmäßigerweise aus einem bioverträglichen Material gebildet. Als Beispiel für bevorzugte Fadenmaterialien sind Polyester, insbesondere Polyethylenterephthalat zu nennen.

Die erfindungsgemäße Aortensinusprothese kann eine Wasserdurchlässigkeit im Bereich von 0 bis 700 oder mehr ml/cm² min, insbesondere im Bereich von 0 bis 500 ml/cm² min, bevorzugt von 200 bis 250 ml/cm² min, aufweisen. Diese Wasserdurchlässigkeitswerte wurden nach der Bestimmungsmethode von Wesolowski bei 120 mmHg gemessen. Auf diese Weise zeigt sich die Aortensinusprothese nach einer kurzen Zeit blutdicht und das Risiko von Sickerblutungen ist gering.

Das Gewebe der erfindungsgemäßen Aortensinusprothese ist insbesondere in einer Dicke von 0,1 bis 0,35 mm, vorzugsweise von 0,15 bis 0,25 mm, ausgebildet. Die Dickenbestimmung erfolgt nach DIN 863.

Bevorzugt ist das Gewebe mit Fäden der Garnstärke 10 bis 200 dtex ausgebildet. Der Garn kann beispielsweise eine Verzwirnung von 50 bis 500 Umdrehungen/m aufweisen. Die Fäden der gewebten Aortensinusprothese können weiterhin insbesondere 10 bis 200 Filamente, insbesondere 20 bis 150 Filamente, vorzugsweise ca. 80 Filamente, pro Faden aufweisen. Erfindungsgemäß kann das Gewebe mit einem Rapport von 8 x 8 ausgebildet sein.

In einer bevorzugten Ausführungsform liegt die Fadendichte der Kettfäden im zylindrischen Prothesenabschnitt bei 30 bis 120 Fäden pro cm Prothesenumfang, insbesondere bei 40 bis 80 Fäden pro cm Prothesenumfang. Die Fadendichte der Schussfäden im zylindrischen Prothesenabschnitt liegt vorzugsweise bei 10 bis 70 Fäden pro cm Prothesenlänge, insbesondere bei 20 bis 50 Fäden pro cm Prothesenlänge. Mit besonderem Vorteil lässt sich die Weichheit der Prothese über die Schussfadendichte einstellen. Je geringer die Schussfadendichte ist, desto weicher ist die Prothese ausgebildet.

Bei einer Ausführung der Erfindung besitzen der erste herzferne Abschnitt und der dritte herznahe Abschnitt voneinander abweichende Innendurchmesser verbunden mit einem entsprechend abweichenden Kettfadenabstand. Der herznahe dritte Abschnitt besitzt vorzugsweise einen größeren Innendurchmesser. Die Innendurchmesser von Gefäßprothesen sind in aufsteigender Reihe der Prothesengröße üblicherweise geradzahlig um jeweils 2 mm größer. Vorzugsweise beträgt auch die Durchmesserdifferenz zwischen dem dritten und dem ersten Abschnitt jeweils 2 mm.

Je nach den anatomischen Verhältnissen kann der herznahe dritte Abschnitt auch einen kleineren Innendurchmesser aufweisen als der herzferne erste Abschnitt, so dass sich die Verhältnisse umkehren. Es ist auch möglich, den ersten und/oder dritten Abschnitt etwas konisch auszubilden.

Der Bulbusabschnitt bzw. Einzelbulbi innerhalb eines Bulbusabschnittes können auf verschiedene Weise ausgebildet werden, wobei auch Kombinationen der verschiedenen Herstellungsarten möglich sind. Bei einer Möglichkeit werden beim Weben eines Schlauchrohlings sogenannte V-Schienen verwendet, die die Abstände der Kettfäden im Bulbusbereich über den gesamten Umfang oder über Umfangsabschnitte (zur Ausbildung mehrerer Einzelbulbi) vergrößern. Bei einer anderen Ausführung sind die Bulbi durch Strecken der Schussfäden ausgebildet. Hierzu eignet sich eine mechanische Durchmesservergrößerung eines vorgebildeten Gewebeschlauches an den Stellen, an denen der Bulbus vorgesehen ist. Durch partielle Durchmessererweiterung entlang des Schlauchumfangs können auch mehrere Einzelbulbi ausgebildet werden.

Zur Ausbildung der erfindungsgemäßen Aortensinusprothese können grundsätzlich alle Standard-Bindungen für Gewebe verwendet werden. Bevorzugt ist die erfindungsgemäße Aortensinusprothese im wesentlichen in Leinwandbindung ausgebildet. Die erfindungsgemäße Aortensinusprothese kann insbesondere ein Gerüst mit Leinwandbindung besitzen.

Mit Vorteil kann das Gewebe aus einer Kombination aus glatten und texturierten Fäden gebildet sein. Beispielsweise kann das Gesamtverhältnis von glatten zu texturierten Fäden bei 1 : 1 bis 6 : 1, insbesondere bei 3 : 1, liegen. In einer besonders bevorzugten Ausführungsform enthält das Gewebe der erfindungsgemäßen Aortensinusprothese mindestens 50% glatte, das heißt nicht texturierte, Fäden.

Bei den Kettfäden handelt es sich bevorzugt nur um glatte Fäden. In einer möglichen Ausführungsform handelt es sich bei den Kettfäden nur um texturierte Fäden. Als Kettfäden können insbesondere in alternierender Folge glatte und texturierte Fäden verwendet sein, wobei das Verhältnis von glatten zu texturierten Fäden im Verhältnis zwischen 2 : 1 und 1 : 2, insbesondere bei ca. 1 : 1, liegt.

In einer weiteren Ausführungsform können die Schussfäden glatt oder texturiert sein. Vorzugsweise handelt es sich bei den Schussfäden um glatte Fäden. In einer anderen Ausführungsform können als Schussfäden insbesondere in wechselnder Folge glatte und texturierte Fäden verwendet sein, wobei das Verhältnis von glatten zu texturierten Fäden im Verhältnis zwischen 5 : 1 und 1 : 5 liegt.

Die Schussfäden liegen im Bereich des Bulbus enger aneinander als im zylindrischen Prothesenbereich. Dies bedeutet, dass die Schussfäden in diesem Bereich vorzugsweise kompaktiert sind. Dies kann durch ein stärkeres Anschlagen der Schussfäden in diesem Bereich bewirkt werden. Somit kann der größere Abstand der Kettfäden durch einen geringeren Abstand der Schussfäden in diesem Bereich kompensiert werden, so dass die Wasserdurchlässigkeit im Vergleich zu den zylindrischen Abschnitten unverändert ist.

Als Schussfäden zumindest für den herzfernen Abschnitt und vorzugsweise auch für den herznahen Abschnitt können mit Vorteil schrumpffähige Fäden, sogenannte Schrumpfgarne, eingesetzt werden. Diese haben vorzugsweise eine absolute Schrumpffähigkeit von mindestens 20 %, vorzugsweise eine Schrumpffähigkeit, die um mindestens 30 %, vorzugsweise mindestens 40 %, höher liegt als eine etwaige Schrumpffähigkeit der Schussfäden des Bulbus, wobei die Schrumpffähigkeit auf nicht schrumpfbare Fäden (mit einem Schrumpf von 0 bis < 5 %) bezogen ist. So kann der Durchmesserunterschied zwischen den im wesentlichen zylindrischen Abschnitten und dem Bulbusabschnitt mindestens zum Teil durch den unterschiedlichen Schrumpf der Schussfäden ausgebildet sein. Auch Durchmesserunterschiede zwischen den im wesentlichen zylindrischen Abschnitten, innerhalb dieser Abschnitte und innerhalb des Bulbus können auf diese Weise erhalten werden.

In besonderen Fällen ist es möglich, dass die Dickenzunahme der Schrumpffäden infolge der Schrumpfung so groß ist, dass eine Ausschrumpfung der Fäden infolge der Kompaktierung der Schussfäden unterbleibt. Ist eine vollständige Ausschrumpfung der Schussfäden erwünscht, dann kann die Dickenzunahme der als Schussfäden verwendeten Schrumpfgarne durch eine geringere Schussfadendichte pro cm Kettrichtung ausgeglichen werden.

Somit bietet die Erfindung geeignete Maßnahmen, das relative Durchmesserverhältnis zwischen Bulbusabschnitt und zylindrischen Abschnitten sowie die Wasserdurchlässigkeit der einzelnen Abschnitte in gewünschter Weise einzustellen.

Die erfindungsgemäße Aortensinusprothese weist bevorzugt zumindest im ersten, zylindrischen Prothesenabschnitt eine Plissierung auf. Auch im Bulbusabschnitt sowie im dritten zylindrischen Prothesenabschnitt ist eine Plissierung möglich. Bevorzugt handelt es sich bei der Plissierung um eine in Querrichtung verlaufende Plissierung.

Mit besonderem Vorteil ist die erfindungsgemäße Aortensinusprothese im Bereich des Bulbusabschnittes in Umfangrichtung dehnbar ausgebildet. Die Aortensinusprothese weist vorzugsweise im Bulbusbereich eine in Längsrichtung verlaufende Plissierung auf. Auf diese Weise kann die Compliance (Dehnbarkeit) der Aortensinusprothese zusätzlich verbessert werden. Plissierfalten der in Längsrichtung verlaufenden Plissierung können thermisch ausgeprägt (fixiert) sein, insbesondere nach außen.

In einer vorteilhaften Ausführungsform der Erfindung können flottierende Fäden, insbesondere in Kettrichtung verlaufende Flottungsfäden, eingebracht sein. Ein derartiger Flottungsfaden ist vorzugsweise zusätzlich zu und insbesondere parallel neben einem Kettfaden in der Leinwandbindung vorhanden. Der zusätzliche Flottungsfaden läuft dort, wo er in Leinwandbindung liegt, vorzugsweise zusammen mit dem parallelen Kettfaden und trifft sich nach der Flottung wieder mit dem Kettfaden. Die Flottungsfäden können in der Gewebefläche flach liegen und insbesondere keine Schlaufen bilden. Mit besonderem Vorteil können die Flottungsfäden texturiert sein. In einer bevorzugten Ausführungsform liegt ein flottierender texturierter Faden in Nachbarschaft zu zwei in gleicher Richtung verlaufenden glatten Fäden des Gewebes in der Gewebebindung.

In einer weiteren Ausführungsform kann das Gewebe der erfindungsgemäßen Aortensinusprothese nur aus glatten Fäden mit einer Flottung aus texturierten Fäden ausgebildet sein. In einer anderen Ausführungsform kann das Gewebe aus abwechselnd glatten und texturierten Fäden mit einer Flottung aus texturierten Fäden ausgebildet sein, wobei insbesondere nur jeder zweite texturierte Faden eine Flottung aufweist.

Die Flottungsfäden verlaufen bevorzugt nur in Kettrichtung. In einer anderen Ausführungsform können die Flottungsfäden sowohl in Kettrichtung als auch in Schussrichtung verlaufen. Die Flottungsfäden können insbesondere in Kettrichtung und in Schussrichtung in unterschiedlicher Zahl eingebracht sein.

Erfindungsgemäß ist es weiterhin bevorzugt, dass die Flottungen über mehr als 2 Fäden verlaufen, insbesondere über 3 bis 10 Fäden, vorzugsweise über 4 bis 6 Fäden. Mit Vorteil kann die Flottung 4 bis 6 über 1, bevorzugt 4 über 2 oder 5 über 1, betragen. In einer bevorzugten Ausführungsform kann bei einem Rapport von 8 x 8 eine Flottung über 5 unter 1 über 1 unter 1 eingebracht sein. Bei der Flottung ist eine ungerade Zahl bevorzugt, da sonst der Flottungsfaden nicht mehr parallel zu einem benachbarten Gewebefaden einer Leinwandbindung liegt.

Gemäss der Erfindung kann das Verhältnis von Flottungsfäden zu Kettfäden zwischen 1 : 20 bis 1 : 1, insbesondere zwischen 1 : 10 bis 1 : 2, bevorzugt bei 1 : 3, liegen. Mit anderen Worten können Flottungsfäden in einem Anteil von etwa 5 bis 50%, insbesondere von 9 bis 33%, bevorzugt von 25%, bezüglich der Kettfäden im Gewebe vorhanden sein. Bevorzugt können Flottungen von benachbarten Flottungsfäden in Kettrichtung versetzt sein, wobei sich vorzugsweise mindestens ein Teil der Flottungen überschneidet.

Mit Vorteil kann der Abstand von Flottungsfäden in Schussrichtung zueinander jeweils gleich sein. Insbesondere können mindestens drei Fäden des Grundgewebes dazwischen liegen. Es können in Schussrichtung maximal zwei Überlegungen von Kettfäden nebeneinander liegen.

Bei der erfindungsgemäßen Aortensinusprothese können die Flottungsfäden nur auf einer Oberfläche flottieren. Vorzugsweise weist die Bindung des Gewebes auf der Außenseite der Aortensinusprothese Flottungen auf, die insbesondere aus texturierten Fäden bestehen. Dadurch können Fibroblasten ausgehend vom umliegenden Gewebe in die Au-ßenseite der Aortensinusprothese einwachsen und das Kollagen für eine äußere Verkapselung der Prothese bilden.

Die Protheseninnenseite ist vorzugsweise frei von Flottungen. Mit besonderem Vorteil besitzt die Innenseite der erfindungsgemäßen Aortensinusprothese eine glattere Oberfläche als außen. Dies kann insbesondere das Einwachsen einer Neointima begünstigen. So können Mesothelzellen ausgehend von Gefässstümpfen eine neu dünne und glatte Neointima bilden.

Durch eine stärkere Heranziehung von glatten Fäden zur Ausbildung der Innenseite der Aortensinusprothese wird eine glatte innere Oberfläche erzeugt. Demgegenüber wird durch eine vorzugsweise stärkere Heranziehung von texturierten Fäden für die Ausbildung der Außenseite der Aortensinusprothese eine Außenseite erzeugt, welche eine verglichen mit der Innenseite voluminöse Struktur aufweist. Durch diese stärker strukturierte, äußere Oberfläche wird ein Porositätsgradient hin zur inneren Oberfläche der erfindungsgemäßen Aortensinusprothese erhalten, wodurch ein verbessertes Einwachsen von Blutgefässen bei gleichzeitig begünstigter Ausbildung der Neointima erzielt wird.

In einer weiteren Ausführungsform ist die erfindungsgemäße Aortensinusprothese abdichtend imprägniert. Zur Imprägnierung werden bevorzugt resorbierbare Materialen verwendet. Als Imprägnierungsmaterialien kommen beispielsweise Gelatine und andere geeignete natürliche oder synthetische Materialien in Betracht. Mit besonderem Vorteil werden die Imprägnierungsmaterialen nach Beschichtung der Aortensinusprothese vernetzt.

Die Aortensinusprothese ist erfindungsgemäß frei von Verbindungsnähten, insbesondere zwischen den Bulbi und anderen Abschnitten.

In einer weiteren geeigneten Ausführungsform weist der Bulbus mehrere, insbesondere drei in der Wandung des Bulbus längsverlaufende, Verstärkungen auf. Bei den Verstärkungen kann es sich insbesondere um Prothesenmaterial, Kunststoffe oder Metalle handeln. Beispielsweise kann es sich bei den Verstärkungen um zusammengerafftes Prothesenmaterial handeln. Weiterhin können die Verstärkungen durch zusätzliches Prothesenmaterial gebildet sein, das beispielsweise auf die Aortensinusprothese aufgenäht wird. Die Verstärkungen sind bevorzugt steg- oder streifenförmig ausgebildet. Durch die Verstärkungen ist mit besonderem Vorteil eine sichere Fixierung der erfindungsgemäßen Aortensinusprothese während des Implantationsvorganges möglich, indem die Stege - die sogenannten Kommissuren - der natürlichen Aortenwand an den Verstärkungen befestigt, insbesondere angenäht, werden. Die Verstärkungen sind bevorzugt als Verstärkungsnähte ausgebildet.

Ferner kann die erfindungsgemäße Aortensinusprothese mit medizinisch wirksamen Substanzen ausgerüstet sein. Als derartige Wirksubstanzen kommen insbesondere Antibiotika, Antiseptika, Gerinnungsfaktoren, Wachstumsfaktoren und dergleichen in Betracht.

Erfindungsgemäß kann die Aortensinusprothese insbesondere durch thermische Fixierung geschrumpft sein. Insbesondere können texturierte Fäden durch die Schrumpfung geöffnet sein.

Die erfindungsgemäße Aortensinusprothese liegt weiterhin vorzugsweise in sterilisierter und insbesondere konfektionierter Form vor.

Weitere Merkmale und Einzelheiten der Erfindung ergeben sich aus der nachfolgenden Beschreibung einer bevorzugten Ausführungsform in Form eines Herstellungsbeispiels. Dabei können die einzelnen Merkmale jeweils für sich alleine oder zu mehreren in Kombination miteinander verwirklicht sein. Das Beispiel dient lediglich als Erläuterung der vorliegenden Erfindung, die in keiner Weise darauf beschränkt sein soll.

### Figurenbeschreibung

In den Zeichnung zeigen
- Figur 1:: eine Seitenansicht einer Ausführungsform der Aortensinusprothese
- Figur 2:: eine Draufsicht des äußersten rechten Kettfadens einer Alternative zur Ausführungsform nach Figur 1
- Figur 3:: einen Querschnitt entlang der Linie III-III nach Figur 1
- Figur 4:: einen Querschnitt entlang der Linie IV-IV nach Figur 1
- Figur 5:: einen Querschnitt eines ungleichmäßig erweiterten Bulbusabschnittes
- Figur 6:: eine Seitenansicht einer anderen Ausführungsform
- Figur 7:: einen Querschnitt einer weiteren Ausführungsform.

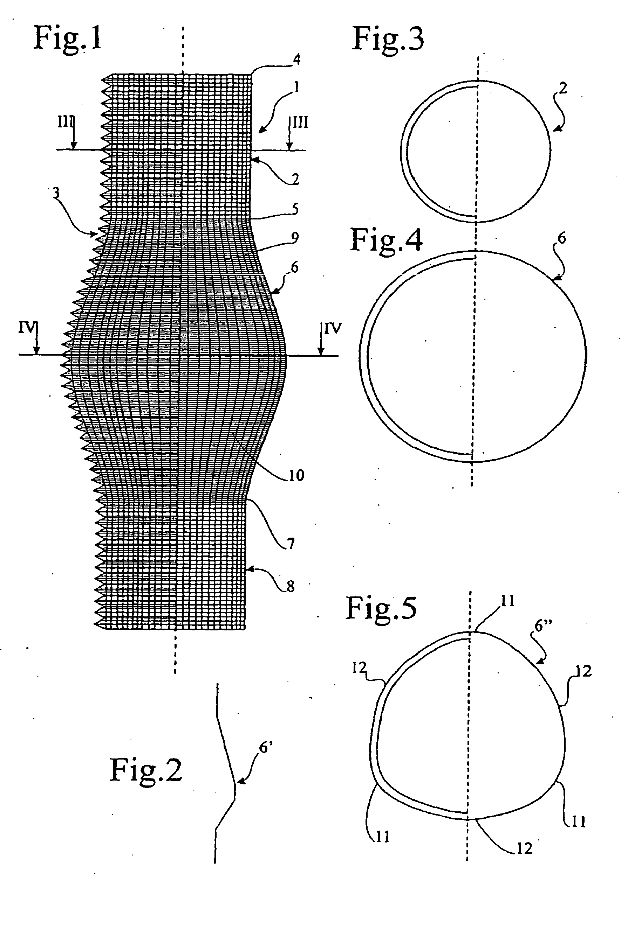

Bei der in der Zeichnung in Figur 1, 3 und 4 dargestellten Ausführungsform weist eine gewebte Aortensinusprothese 1 einen herzfernen Abschnitt 2 auf, der abgesehen von einer Querplissierung 3 zylindrisch ausgebildet ist. Der herzferne Abschnitt 2 kann an seinem oberen Ende 4 nahtlos oder mittels einer Quernaht in einen in der Zeichnung nicht dargestellten Aortenbogen übergehen. An seinem unteren Ende 5 geht der herzferne Abschnitt 2 der Aortensinusprothese axial und nahtlos in einen Bulbusabschnitt 6 über. Der Bulbusabschnitt 6 kann in Umfangsrichtung betrachtet gleichmäßig (Fig.1 und Fig.4) oder ungleichmäßig (Fig. 5) erweitert sein. Auch der Bulbusabschnitt 6 weist eine Querplissierung 3 auf. An das untere Ende 7 des Bulbusabschnittes 6 schließt sich wiederum axial und nahtlos ein im wesentlichen zylindrischer herznaher Abschnitt 8 an, der wiederum abgesehen von der Querplissierung 3 zylindrisch ausgebildet ist. Die Querplissierung 3 erstreckt sich somit über die gesamte Länge der Aortensinusprothese 1.

Bei der Ausführungsform nach Figur 1 weisen die Kettfäden 9 im Bulbusabschnitt 6 einen allmählich zunehmenden und wieder abnehmenden größeren Abstand voneinander auf als in den zylindrischen Abschnitten 2 und 8. Dadurch ergibt sich ein ballonartiger Bulbus 6. Zudem liegen bei dieser Ausführungsform die Schussfäden 10 im Bulbusabschnitt 6 enger aneinander als in den zylindrischen Bereichen der Aortensinusprothese 1.
Beim Bulbus 6' der Ausführungsform nach Figur 2 ist die Zunahme der Abstände der Kettfäden zunächst gleichmäßig, so dass der Bulbusdurchmesser linear ansteigt. Der Durchmesser bleibt dann über eine vorbestimmte Länge konstant und dann wieder linear aber steiler ab als bei der Zunahme. Bei der Ausführungsform nach Figur 5 weist ein Bulbus 6" drei Umfangsabschnitte 11 auf, bei denen die Abstände der Kettfäden stark erweitert sind, und drei dazwischen liegende Umfangsabschnitte 12, bei denen die Abstände der Kettfäden weniger stark erweitert sind. Dadurch werden drei Einzelbulbi 11 ausgeformt oder zumindest angedeutet.

Der Bulbusabschnitt 6 kann eine natürliche oder auch eine künstliche Herzklappe aufnehmen. Der untere zylindrische Abschnitt 8 kann außerdem verkürzt sein oder bei Bedarf abgeschnitten werden, je nachdem wie viel vom natürlichen Aortenansatz (Annulus) bei der Implantation stehen bleibt.

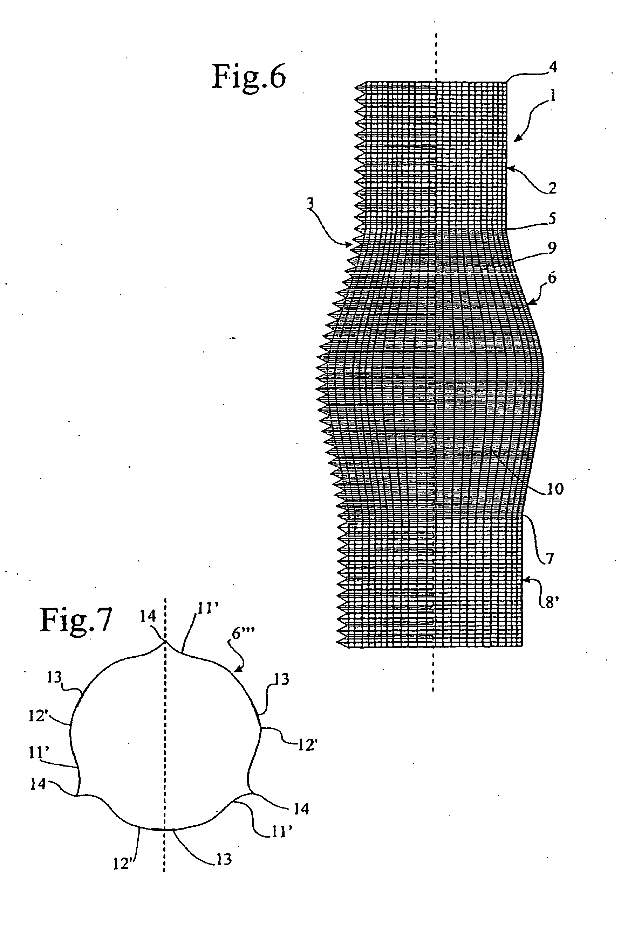

Bei der Ausführungsform nach Figur 6 sind die drei Abschnitte im wesentlichen gleich ausgebildet wie bei der Ausführungsform nach Figur 1. Deshalb werden entsprechende Bezugszahlen verwendet. Der Unterschied zur Ausführungsform nach Figur 1 besteht darin, dass der herznahe Abschnitt 8' einen größeren Innendurchmesser besitzt als der erste herzferne Abschnitt 2. Die Durchmesserdifferenz beträgt bevorzugt 2 mm. Wenn also der erste herzferne Abschnitt einen Innendurchmesser von beispielsweise 28 mm besitzt, dann beträgt der Innendurchmesser des herznahen dritten Abschnittes 8' 30 mm. Die Zahl der Kettfäden bleibt wiederum unverändert. Lediglich der Kettfadenabstand ist im herznahen dritten Abschnitt größer als im herzfernen ersten Abschnitt. Die Durchmesserdifferenz beträgt auch bei größeren oder kleineren Aortensinusprothesen mit größeren oder kleineren Innendurchmessern vorzugsweise jeweils ca. 2 mm.

### Herstellungsbeispiel

Auf einem Webstuhl wird eine schlauchförmige Prothese mit einem Durchmesser von 28 mm in Leinwandbindung gewebt. Die so hergestellte Prothese besitzt über eine axiale Länge von ca. 30 mm einen Bulbus (Aussackung) mit einem Durchmesser von ca. 32 mm. Der Bulbus kann durch Verwendung von V-Schienen beim Weben ausgebildet werden, die eine Spreizung der Kettfäden im Bulbusbereich bewirken. Oberhalb und unterhalb des Bulbus besitzt die hergestellte Prothese einen Durchmesser von ca. 28 mm. Die Prothese kann als beliebig langer Schlauch gewebt werden, bei dem sich die Bulbusabschnitte in vorbestimmten Abständen wiederholen. Durch Abschneiden zwischen den Bulbusabschnitten werden die Prothesenrohlinge erhalten. Für die Herstellung der Prothese können Schussfäden mit unterschiedlich starker Schrumpffähigkeit eingesetzt werden. Während für den Bulbus verwendete Schussfäden nicht oder nur wenig schrumpffähig sind, können für die im wesentlichen zylindrischen Abschnitte Schrumpfgarne eingesetzt werden, die eine Schrumpffähigkeit von mindestens 30 % besitzen. Solche Schrumpfgarne sind bekannt und können einen Schrumpf bis 70 % besitzen. Durch entsprechende Auswahl solcher Schrumpfgarne können allmähliche oder abrupte Durchmesserunterschiede bei der Prothese ausgebildet oder verstärkt werden. Der Schrumpf wird insbesondere durch eine thermische Behandlung der gewebten Prothese erzeugt. Beim Schrumpfen der Garne findet eine Erhöhung der Garndurchmesser statt. Falls eine dadurch bedingte Kompaktierung der Schussfäden unerwünscht ist, kann diese durch eine geringere Schussfadendichte in Kettrichtung kompensiert werden. Durch eine geringere Schussfadendichte kann allgemein eine höhere Prothesenweichheit erzielt werden.

Die Prothesenrohlinge werden nach ihrer Herstellung oberhalb des Bulbus und gegebenenfalls unterhalb des Bulbus plissiert. Die plissierten Bereiche werden anschließend auf ca. 10 cm und ca. 3 cm Länge zurechtgeschnitten. Danach werden in den unplissierten Bulbus durch Zusammenraffen des Prothesenmaterials in Längsrichtung der Prothese drei Stege 13 (Figur 7) als Verstärkungen in gleichem Abstand zueinander mit einer Breite von ca. 3 mm eingenäht. Nach Ausbildung der Verstärkungen im Bulbusbereich wird die Prothese mit Gelatine beschichtet. Die Gelatine wird vernetzt. Schließlich wird die Prothese einer γ-Sterilisation unterworfen. Zusätzlich oder anstelle der Verstärkungen 13 kann die Prothese im Bulbusbereich mit einer Längsplissierung versehen werden. So sind insbesondere drei Längsplissierfalten 14 vorgesehen. Diese können mit Vorteil an den Stellen der erweiterten Umfangsabschnitte 11 (Figur 5) in Form von drei thermisch fixierten nach außen ragenden Längsfalten ausgebildet sein, wie dies in Figur 7 schematisch dargestellt ist. Mit Vorteil können anstelle der üblichen zwei Guidelinelinien drei im Umfang um 120° gegeneinander versetzte Guidelinelinien als Führungslinien vorgesehen sein.

Eine weitere Möglichkeit, Bulbusabschnitte auszubilden, besteht darin, eine mechanische Durchmesservergrößerung an einer zylindrischen Schlauchprothese oder an einer Prothese mit vorgebildeten Bulbi vorzunehmen. Hierzu eignet sich eine mechanische Aufweiteinrichtung, insbesondere ein zylindrischer Aufweitdorn, der im Bulbusbereich radial nach außen drückbare Nocken besitzt. Bei der mechanischen Aufweitung werden die Schussfäden gestreckt, insbesondere kalt gestreckt. Durch thermische Behandlung im gestreckten Zustand können die aufgeweiteten Bulbi fixiert werden. Die Prothese kann vor der mechanischen Aufweitung bereits plissiert sein.

## Patentansprüche

1. Gewebte Aortensinusprothese (1) mit einem ersten, herzfernen im Wesentlichen zylindrischen Abschnitt (2), der gegebenenfalls in einen Aortenbogen übergeht, einem zweiten, im Vergleich zum ersten Abschnitt (2) im Durchmesser erweiterten, einen Bulbus bildenden Abschnitt (6), der sich an den ersten Abschnitt (2) anschließt, und einem dritten, im Wesentlichen zylindrischen, herznahen Abschnitt (8), der sich an den Bulbusabschnitt (6) anschließt, **dadurch gekennzeichnet, dass** die Prothese (1) durchgehend gewebt ist, frei von Verbindungsnähten ist und über ihre axiale Länge eine konstante Zahl an Kettfäden (9) aufweist, wobei Kettfäden (9) im Bereich des Bulbusabschnittes (6) einen größeren Abstand voneinander haben als in zylindrischen Bereichen (2; 8) und die Schussfäden (10) im Bereich des Bulbusabschnittes (6) enger aneinander liegen als in den zylindrischen Prothesenbereichen (2; 8) und die Kettfäden (9) im Bulbusabschnitt (6) im Querschnitt unterschiedlich große Abstände voneinander besitzen.

2. Gewebte Aortensinusprothese (1) nach Anspruch 1, **dadurch gekennzeichnet, dass** der Bulbusabschnitt (6) einen maximalen Durchmesser aufweist, der 1 bis 45 %, vorzugsweise 10 bis 40 %, größer ist als der Durchmesser von zylindrischen Abschnitten (2; 8).

3. Gewebte Aortensinusprothese (1) nach Anspruch 1 oder 2, **dadurch gekennzeichnet, dass** der Bulbus im Abschnitt (6) mindestens zum Teil durch mechanische Aufweitung des Gewebes, insbesondere von Schussfäden, im Bulbusbereich gebildet ist, und das aufgeweitete Gewebe in aufgeweitetem Zustand thermisch fixiert ist.

4. Gewebte Aortensinusprothese (1) nach einem der vorhergehenden Ansprüche, **dadurch gekennzeichnet, dass** der Bulbusabschnitt (6) in axialer Richtung drei Längsunterabschnitte besitzt, von denen ein erster, herzferner Unterabschnitt eine stetige, insbesondere kontinuierliche Abstandsvergrößerung der Kettfäden (9) aufweist, ein zweiter, mittlerer Unterabschnitt im wesentlichen konstante Abstände der Kettfäden (9) aufweist und ein dritter, herznaher Unterabschnitt eine stetige Verjüngung der Abstände der Kettfäden (9) aufweist bis zum Durchmesser des zylindrischen Prothesenabschnittes (8).

5. Gewebte Aortensinusprothese (1) nach Anspruch 4, **dadurch gekennzeichnet, dass** die drei Bulbusunterabschnitte in axialer Richtung verschieden lang ausgebildet sind.

6. Gewebte Aortensinusprothese (1) nach Anspruch 4 oder 5, **dadurch gekennzeichnet, dass** der erste Bulbusunterabschnitt mindestens gleich lang ist, vorzugsweise länger ist als der dritte Unterabschnitt.

7. Gewebte Aortensinusprothese (1) nach einem der vorhergehenden Ansprüche, **dadurch gekennzeichnet, dass** die Fadendichte der Kettfäden (9) in den zylindrischen Prothesenabschnitten (2; 8) bei 30 bis 120 Fäden pro cm Prothesenumfang liegt, insbesondere von 40 bis 80 Fäden pro cm.

8. Gewebte Aortensinusprothese (1) nach einem der vorhergehenden Ansprüche, **dadurch gekennzeichnet, dass** der erste, herzferne Abschnitt (2) und der dritte, herznahe Abschnitt (8) voneinander abweichende Innendurchmesser und voneinander abweichende Kettfädenabstände besitzen, wobei der dritte, herznahe Abschnitt (8) vorzugsweise einen größeren Innendurchmesser besitzt als der erste herzferne Abschnitt (2).

9. Gewebte Aortensinusprothese (1) nach einem der vorhergehenden Ansprüche, **dadurch gekennzeichnet, dass** die Fadendichte der Schussfäden (10) in den zylindrischen Prothesenabschnitten (2; 8) bei 10 bis 70 Fäden pro cm Prothesenlänge liegt, insbesondere von 20 bis 50 Fäden pro cm.

10. Gewebte Aortensinusprothese (1) nach einem der vorhergehenden Ansprüche, **dadurch gekennzeichnet, dass** der herzferne Abschnitt, und vorzugsweise auch der herznahe Abschnitt, unter Verwendung von schrumpffähigen Schussfäden mit einer Schrumpffähigkeit von vorzugsweise mindestens 20 % hergestellt ist und der Durchmesserunterschied im Vergleich zum Bulbusabschnitt mindestens zum Teil durch Schrumpf dieser Fäden bewirkt ist.

11. Gewebte Aortensinusprothese (1) nach einem der vorhergehenden Ansprüche, **dadurch gekennzeichnet, dass** die Schrumpffähigkeit im herzfernen und vorzugsweise auch im herznahen Abschnitt um mindestens 30 %, vorzugsweise um mindestens 40 %, bezogen auf nicht schrumpfbare Fäden, größer ist als eine etwaige Schrumpffähigkeit der im Bulbusbereich verwendeten Schussfäden.

12. Gewebte Aortensinusprothese (1) nach einem der Ansprüche 10 bis 11, **dadurch gekennzeichnet, dass** die Schussdichte bei Verwendung von schrumpffähigen Schussfäden geringer eingestellt ist als die Schussdichte von normalen (nicht schrumpffähigen) Schussfäden, vorzugsweise in dem Maße, in dem beim Schrumpf eine Dickenzunahme der Schrumpfgarne erfolgt.

13. Gewebte Aortensinusprothese (1) nach einem der vorhergehenden Ansprüche, **dadurch gekennzeichnet, dass** die Prothese (1) zumindest im ersten, zylindrischen Prothesenabschnitt (2), vorzugsweise nur im ersten zylindrischen Prothesenabschnitt (2) eine Plissierung aufweist.

14. Gewebte Aortensinusprothese (1) nach einem der vorhergehenden Ansprüche, **dadurch gekennzeichnet, dass** die Prothese (1) im Bereich des Bulbusabschnittes (6) in Umfangsrichtung dehnbar ausgebildet ist, insbesondere eine in Längsrichtung verlaufende Plissierung aufweist.

15. Gewebte Aortensinusprothese (1) nach einem der vorhergehenden Ansprüche, **dadurch gekennzeichnet, dass** Plissierfalten der in Längsrichtung verlaufenden Plissierung durch thermische Prägung ausgebildet sind.

16. Gewebte Aortensinusprothese (1) nach einem der vorhergehenden Ansprüche, **dadurch gekennzeichnet, dass** die Bindung des Gewebes auf der Außenseite Flottungen aufweist, und auf der Protheseninnenseite frei von Flottungen ist.

17. Gewebte Aortensinusprothese (1) nach einem der vorhergehenden Ansprüche **dadurch gekennzeichnet, dass** die Gefäßprothese (1) abdichtend imprägniert ist.

18. Gewebte Aortensinusprothese (1) nach einem der vorhergehenden Ansprüche, **dadurch gekennzeichnet, dass** der Bulbusabschnitt (6) mehrere, insbesondere drei in der Wandung des Bulbusabschnittes (6) längs verlaufende Verstärkungen aufweist.

19. Gewebte Aortensinusprothese (1) nach Anspruch 18, **dadurch gekennzeichnet, dass** die Verstärkungen als Verstärkungsnähte ausgebildet sind.

20. Gewebte Aortensinusprothese (1) nach Anspruch 1, **dadurch gekennzeichnet, dass** der Bulbusabschnitt (6) drei Einzelbulbi aufweist, die durch drei Umfangsbereiche mit vergrößerten Abständen von Kettfäden (9) gebildet werden.

## Claims

1. Woven aortic sinus prosthesis (1) having a first substantially cylindrical section (2) not close to the heart, which may possibly pass into an aortic arch, a second section (6) having a greater diameter as compared to the first section (2) and forming a bulb, which second section is connected to the first section (2), and a third, substantially cylindrical section (8) close to the heart, which is connected to the bulb section (6), **characterized in that** the prosthesis (1) is continuously woven, is free of joining seams and has a constant number of warp yarns (9) over its axial length, wherein warp yarns (9) in the region of the bulb section (6) have a greater distance between each other than in the cylindrical regions (2; 8), and the weft yarns (10) in the region of the bulb section (6) lie closer together than in the cylindrical regions of the prosthesis (2; 8), and the warp yarns (9) in the bulb section (6) are a different distance apart in the cross section.

2. Woven aortic sinus prosthesis (1) according to Claim 1, **characterized in that** the bulb section (6) has a maximum diameter, which is 1 to 45 %, preferably 10 to 40 %, greater than the diameter of cylindrical sections (2; 8).

3. Woven aortic sinus prosthesis (1) according to Claim 1 or 2, **characterized in that** the bulb in the section (6) is formed at least partially by mechanical widening of the woven fabric, in particular of weft yarns, in the bulb region, and the widened woven fabric is fixed thermally in the widened state.

4. Woven aortic sinus prosthesis (1) according to one of the previous claims, **characterized in that** the bulb section (6) has three longitudinal sub-sections in the axial direction, in which the distance between the warp yarns (9) of a first sub-section, not close to the heart increases constantly, in particular continuously, a second central sub-section, in which the distance between the warp yarns (9) is substantially constant, and a third sub-section close to the heart, in which the distance between the warp yarns (9) constantly tapers up to the diameter of the cylindrical section of the prosthesis (8).

5. Woven aortic sinus prosthesis (1) according to Claim 4, **characterized in that** the three bulb sub-sections have different lengths in the axial direction.

6. Woven aortic sinus prosthesis (1) according to Claim 4 or 5, **characterized in that** the first bulb sub-section is at least as long as, and preferably longer than, the third sub-section.

7. Woven aortic sinus prosthesis (1) according to one of the previous claims, **characterized in that** the yarn density of the warp yarns (9) in the cylindrical sections of the prosthesis (2; 8) is 30 to 120 yarns per cm of the prosthesis circumference, particularly 40 to 80 yarns per cm.

8. Woven aortic sinus prosthesis (1) according to one of the previous claims, **characterized in that** the first section (2) not close to the heart and the third section (8) close to the heart have different internal diameters and different warp yarn spacings, wherein the third section (8) close to the heart preferably has a greater internal diameter than the first section (2) not close to the heart.

9. Woven aortic sinus prosthesis (1) according to one of the previous claims, **characterized in that** the yarn density of the weft yarns (10) in the cylindrical sections of the prosthesis (2; 8) is 10 to 70 yarns per cm of the prosthesis length, particularly 20 to 50 yarns per cm.

10. Woven aortic sinus prosthesis (1) according to one of the previous claims, **characterized in that** the section not close to the heart, and preferably also the section close to the heart, are produced using shrinkable weft yarns having a shrinkability of preferably at least 20 %, and the difference in diameter compared to the bulb section is at least partly realized by the shrinkage of these yarns.

11. Woven aortic sinus prosthesis (1) according to one of the previous claims, **characterized in that** the shrinkability in the section not close to the heart and preferably also in the section close to the heart is at least 30 %, preferably at least 40 %, higher than a possible shrinkability of the weft yarns used in the bulb region, in relation to non-shrinkable yarns.

12. Woven aortic sinus prosthesis (1) according to either of Claims 10 and 11, **characterized in that** the weft density is set to be lower if shrinkable weft yarns are used than the weft density of normal (non-shrinkable) weft yarns, preferably to the same extent as an increase in the thickness of the shrink yarns takes place during the shrinking.

13. Woven aortic sinus prosthesis (1) according to one of the previous claims, **characterized in that** the prosthesis (1) has pleats at least in the first cylindrical section of the prosthesis (2), preferably only in the first cylindrical section of the prosthesis (2).

14. Woven aortic sinus prosthesis (1) according to one of the previous claims, **characterized in that** the prosthesis (1) is formed so as to be extensible in the circumferential direction in the region of the bulb section (6), in particular has pleats running in the longitudinal direction.

15. Woven aortic sinus prosthesis (1) according to one of the previous claims, **characterized in that** the pleated folds of the pleats running in the longitudinal direction are formed by thermal embossing.

16. Woven aortic sinus prosthesis (1) according to one of the previous claims, **characterized in that** the woven fabric construction has floats on the outer side and is free of floats on the inner side of the prosthesis.

17. Woven aortic sinus prosthesis (1) according to one of the previous claims, **characterized in that** the vascular prosthesis (1) has a sealing impregnation.

18. Woven aortic sinus prosthesis (1) according to one of the previous claims, **characterized in that** the bulb section (6) has several, particularly three, reinforcing elements running longitudinally in the wall of the bulb section (6).

19. Woven aortic sinus prosthesis (1) according to Claim 18, **characterized in that** the reinforcing elements are formed as reinforcing seams.

20. Woven aortic sinus prosthesis (1) according to Claim 1, **characterized in that** the bulb section (6) has three individual bulbs, which are formed by three circumferential regions having increased warp yarn (9) spacings.

## Revendications

1. Prothèse tissée (1) du sinus de l'aorte, qui présente un premier tronçon (2) essentiellement cylindrique, éloigné du coeur, qui se prolonge éventuellement en un arc aortique, un deuxième tronçon (6) dont le diamètre est plus grand que celui du premier tronçon (2), formant un bulbe et raccordé au premier tronçon (2), et un troisième tronçon (8) essentiellement cylindrique, proche du coeur, qui se raccorde au tronçon (6) en bulbe,
**caractérisée en ce que**
la prothèse (1) est tissée de manière continue, ne présente pas de couture de liaison et présente sur sa longueur axiale un nombre constant de fils de chaîne (9),
dans la zone occupée par le tronçon (6) en bulbe, les fils de chaîne (9) présentant une plus grande distance mutuelle que dans les parties cylindriques (2; 8) et, dans la zone occupée par le tronçon (6) en bulbe, les fils de trame (10) étant disposés plus près les uns des autres que dans les parties cylindriques (2; 8) de la prothèse et, en coupe transversale, les fils de chaîne (9) présentant dans le tronçon (6) en bulbe des distances mutuelles différentes.

2. Prothèse tissée (1) du sinus de l'aorte selon la revendication 1, **caractérisée en ce que** le tronçon (6) en bulbe présente un diamètre maximum de 1 à 45 % et de préférence de 10 à 40 % plus grand que le diamètre des tronçons cylindriques (2; 8).

3. Prothèse tissée (1) du sinus de l'aorte selon les revendications 1 ou 2, **caractérisée en ce que** le bulbe du tronçon (6) est formé au moins en partie par étirage mécanique du tissu, en particulier des fils de trame, dans la zone occupée par le bulbe et **en ce que** le tissu étiré est fixé thermiquement dans sa position étirée.

4. Prothèse tissée (1) du sinus de l'aorte selon l'une des revendications précédentes, **caractérisée en ce que** le tronçon (6) en bulbe présente dans la direction axiale trois sous-tronçons longitudinaux dont un premier sous-tronçon éloigné du coeur présente un agrandissement constant et en particulier continu de la distance entre les fils de chaîne (9), un deuxième sous-tronçon central qui présente une distance essentiellement constante entre les fils de chaîne (9) et un troisième sous-tronçon proche du coeur qui présente un rétrécissement constant des distances entre les fils de chaîne (9) jusqu'au diamètre du tronçon cylindrique (8) de la prothèse.

5. Prothèse tissée (1) du sinus de l'aorte selon la revendication 4, **caractérisée en ce que** les trois sous-tronçons de bulbe ont des longueurs différentes dans la direction axiale.

6. Prothèse tissée (1) du sinus de l'aorte selon les revendications 4 ou 5, **caractérisée en ce que** le premier sous-tronçon de bulbe a une longueur au moins égale et de préférence plus grande que le troisième sous-tronçon.

7. Prothèse tissée (1) du sinus de l'aorte selon l'une des revendications précédentes, **caractérisée en ce que** la densité de fils de chaîne (9) dans les tronçons cylindriques (2; 8) de la prothèse est de l'ordre de 30 à 120 fils par cm et en particulier de 40 à 80 fils par cm de circonférence de la prothèse.

8. Prothèse tissée (1) du sinus de l'aorte selon l'une des revendications précédentes, **caractérisée en ce que** le premier tronçon (2) éloigné du coeur et le troisième tronçon (8) proche du coeur ont des diamètres intérieurs différents l'un de l'autre et des distances différentes entre les fils de chaîne et **en ce que** le troisième tronçon (8) proche du coeur présente de préférence un diamètre intérieur plus grand que le premier tronçon (2) éloigné du coeur.

9. Prothèse tissée (1) du sinus de l'aorte selon l'une des revendications précédentes, **caractérisée en ce que** la densité des fils de trame (10) dans les tronçons cylindriques (2; 8) de la prothèse est de l'ordre de 10 à 70 fils par cm de longueur de prothèse et en particulier de 20 à 50 fils par cm.

10. Prothèse tissée (1) du sinus de l'aorte selon l'une des revendications précédentes, **caractérisée en ce que** le tronçon éloigné du coeur et de préférence aussi le tronçon proche du coeur sont réalisés en recourant à des fils de trame rétrécissables dont la capacité de rétrécissement est de préférence d'au moins 20 % et **en ce que** la différence de diamètre par rapport au tronçon en bulbe est obtenue au moins en partie par rétrécissement de ces fils.

11. Prothèse tissée (1) du sinus de l'aorte selon l'une des revendications précédentes, **caractérisée en ce que** par rapport aux fils non rétrécissables, la capacité de rétrécissement dans le tronçon éloigné du coeur et de préférence également dans le tronçon proche du coeur est d'au moins 30 % et de préférence d'au moins 40 % plus grande que l'éventuelle capacité de rétrécissement des fils de trame utilisés dans la partie formant le bulbe.

12. Prothèse tissée (1) du sinus de l'aorte selon l'une des revendications 10 à 11, **caractérisée en ce que** lorsque l'on utilise des fils de trame rétrécissables, la densité de fils de trame est réglée à une valeur plus petite que la densité de fils de trame normaux (non rétrécissables), de préférence dans la même mesure que l'augmentation d'épaisseur associée au rétrécissement des fils rétrécissables.

13. Prothèse tissée (1) du sinus de l'aorte selon l'une des revendications précédentes, **caractérisée en ce que** la prothèse (1) présente un plissage au moins dans le premier tronçon cylindrique (2) de la prothèse et de préférence uniquement dans le premier tronçon cylindrique (2) de la prothèse.

14. Prothèse tissée (1) du sinus de l'aorte selon l'une des revendications précédentes, **caractérisée en ce que** la prothèse (1) est extensible dans la direction périphérique dans la zone occupée par le tronçon (6) en bulbe et présente un plissage qui s'étend en particulier dans le sens de la longueur.

15. Prothèse tissée (1) du sinus de l'aorte selon l'une des revendications précédentes, **caractérisée en ce que** les plis du plissage qui s'étend dans le sens de la longueur sont formés par gaufrage thermique.

16. Prothèse tissée (1) du sinus de l'aorte selon l'une des revendications précédentes, **caractérisée en ce que** l'armure du tissu présente un flottage sur le côté extérieur, le côté intérieur de la prothèse étant exempt de flottage.

17. Prothèse tissée (1) du sinus de l'aorte selon l'une des revendications précédentes, **caractérisée en ce que** la prothèse vasculaire (1) est imprégnée de manière à devenir étanche.

18. Prothèse tissée (1) du sinus de l'aorte selon l'une des revendications précédentes, **caractérisée en ce que** le tronçon (6) en bulbe présente plusieurs et en particulier trois renforts qui s'étendent longitudinalement dans la paroi du tronçon (6) en bulbe.

19. Prothèse tissée (1) du sinus de l'aorte selon la revendication 18, **caractérisée en ce que** les renforts sont configurés comme coutures de renfort.

20. Prothèse tissée (1) du sinus de l'aorte selon la revendication 1, **caractérisée en ce que** le tronçon (6) en bulbe présente trois bulbes distincts formés par trois parties périphériques dans lesquelles les distances entre les fils de chaîne (9) sont plus grandes.
